# FASCICULE DE BREVET EUROPEEN

(11) **EP 2 425 863 B1**
(45) Date de publication et mention de la délivrance du brevet: **25.01.2017**
(21) Numéro de dépôt: 11306091.7
(22) Date de dépôt: 02.09.2011
(51) Int. Cl.: A61L 2/26, A61J 1/00, B65D 81/18, B65D 85/00, A61B 50/00

(54) **Emballage pour la stérilisation à la vapeur de produits**
Verpackung für die Dampfsterilisation von Produkten
Packaging for steam sterilisation of products

(30) Priorité: 03.09.2010 FR 1056999
(43) Date de publication de la demande: 07.03.2012
(73) Titulaire: Veriplast Flexible, 43290 Montfaucon en Velay (FR)
(72) Inventeur: Alaux, Patrick, 42240 UNIEUX (FR)
(74) Mandataire: Cabinet Laurent & Charras

(56) Documents cités:
- WO-A1-98/46498
- WO-A2-00/23331
- WO-A2-2005/073091

## Description

L'invention se rattache au secteur technique de l'emballage.

Plus particulièrement, l'invention concerne les emballages utilisés pour la stérilisation des produits et trouve une application particulièrement avantageuse, qui ne saurait toutefois être considérée comme limitative, pour la stérilisation à la vapeur d'un instrument chirurgical en milieu hospitalier.

Après une intervention chirurgicale, le ou les instruments, qui sont souillés, sont mis à tremper dans une solution détergent - désinfectant, pendant une durée déterminée. Le ou les instruments sont ensuite mis dans des paniers métalliques disposés dans un laveur pendant un cycle préalablement établi. A la sortie du laveur, le ou les instruments qui est ou sont sec(s) est ou sont disposé(s) dans des emballages en vue d'être passés à l'autoclave pour un cycle de stérilisation à la vapeur. Ces emballages sont de conception déterminée pour laisser passer la vapeur. Le ou les instruments est ou sont alors prêt(s) pour une nouvelle utilisation.

L'invention concerne plus particulièrement les emballages pour la stérilisation d'instruments chirurgicaux ou autres.

Généralement, ces emballages se présentent sous forme d'enveloppes ou sachets réalisés dans un papier spécial, ou d'enveloppes en polypropylène non tissé. Les emballages, sous forme d'enveloppes ou de sachets en papier, sont relativement fragiles, tandis que ceux en polypropylène non tissé et micro-perforé pour laisser passer la vapeur, présentent un coût plus élevé. Un tel sachet est décrit par exemple dans le brevet WO2005/073091 A2.

Enfin et surtout, les emballages qui se présentent sous forme de sachets ou d'enveloppes, sont limités en capacité et ne permettent pas de recevoir des produits de grandes dimensions, de l'ordre de 30 à 50 cm et d'épaisseur également conséquente de l'ordre de 7 cm, et dont le poids peut aller de 10 à 15 kg, par exemple.

L'invention s'est fixée pour but de remédier à ces inconvénients d'une manière simple, sûre, efficace et rationnelle.

Le problème que se propose de résoudre l'invention est de proposer un emballage destiné à la stérilisation de produits à la vapeur de capacité et de résistance importante et d'un coût de fabrication réduit, avec des cadences de production importante, en utilisant notamment des films généralement utilisés dans le domaine de la fabrication des sacs en matière plastique, avec pour objectif d'obtenir une contenance importante.

Pour résoudre un tel problème, il a été conçu un emballage pour la stérilisation de produits, constitué par un sac en matière plastique conforme aux caractéristiques de la revendication 1.

Dans une forme de réalisation, la paroi apte à laisser passer la vapeur, est fixée par une ligne de soudure déterminée pour permettre la pelabilité de ladite paroi.

Selon une autre caractéristique, la partie ouverte du sac, considérée à l'opposé de son fond, est fermée d'une manière étanche, après mise en place du ou desdits produit(s) à l'intérieur dudit sac.

A partir de cette conception de base de l'emballage, soit la paroi, apte à laisser passer la vapeur, est du papier, soit la paroi, apte à laisser passer la vapeur, est un non tissé.

L'invention est exposée ci-après plus en détail à l'aide des figures des dessins annexés dans lesquels :
- la figure 1 est une vue en perspective de l'emballage avant fixation de la paroi apte à laisser passer la vapeur, au niveau des bords latéraux du sac en matière plastique, notamment au niveau des soufflets ;
- la figure 2 est une vue correspondant à la figure 1 après fixation de la paroi apte à laisser passer la vapeur ;
- la figure 3 est une vue en coupe transversale de l'emballage et dont les épaisseurs des éléments constitutifs ont été exagérées pour une meilleure compréhension des dessins ;
- la figure 4 est une vue en coupe longitudinale de l'emballage ;
- la figure 5 est une vue correspondant à la figure 3 après mise en place d'un produit à l'intérieur de l'emballage.

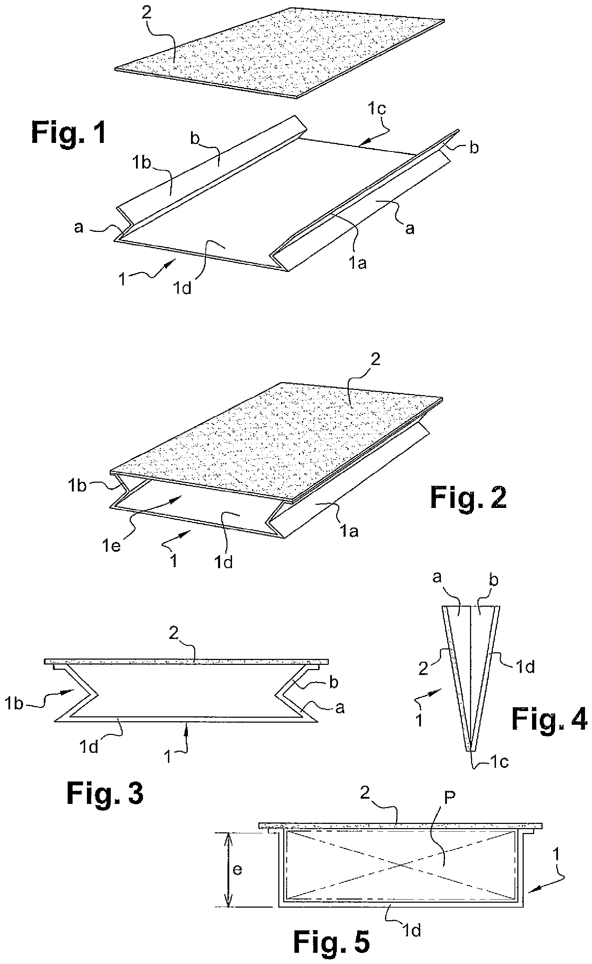

Comme le montrent les figures 1 et 2 notamment, l'emballage pour la stérilisation selon l'invention est constitué par un sac en matière plastique (1) dont l'une des parois au moins est réalisée dans une matière apte à laisser passer la vapeur pendant un cycle de stérilisation. D'une manière parfaitement connue pour un homme du métier, le sac (1) est, par exemple, obtenu à partir d'une bobine de film en matière plastique, lequel film est soumis, en continu, à différentes opérations (coupe, pliage, ...) réalisées en automatique. La matière plastique utilisée peut être, par exemple du PE additionné d'OPP ou de PET, sans pour cela exclure d'autres matières couramment utilisées dans le domaine de l'emballage.

La paroi du sac, réalisée dans une matière apte à laisser passer la vapeur, est constituée par une feuille rapportée (2). Cette feuille (2) est réalisée en papier ou en non tissé, du type de ceux couramment utilisé dans le domaine de la stérilisation à la vapeur.

Cette feuille (2) est rapportée et fixée sur les bords latéraux (1a) et (1b), et l'un des bords transversaux (1c) de l'autre paroi (1d) en matière plastique du sac (1), en constituant le fond. Selon l'invention, les bords latéraux (la) et (1b) sont conformés, notamment par pliage, pour constituer des soufflets formés, d'une manière parfaitement connue, par deux plis symétriques, identiques (a) et (b). La feuille (2) est, par conséquent, fixée sur les bords latéraux des soufflets considérés, du côté du pli (b), et sur le bord transversal (1c) constituant le fond.

Comme le montre notamment la figure 3, la feuille (2) est dimensionnée pour déborder de l'un des côtés au moins du sac pour constituer une zone de préhension en vue de sa pelabilité. Par exemple, la feuille (2) déborde de l'un des côtés latéraux en soufflets du sac (1).

La fixation, en tant que telle, de la feuille (2) s'effectue, là encore d'une manière connue, par une ligne de soudure conformée pour assurer une fixation sure et efficace, tout en permettant sa pelabilité.

Après mise en place du ou des produits (P) à l'intérieur du sac (1), selon les caractéristiques de l'invention (figure 2), ce dernier est fermé d'une manière étanche au niveau de l'ouverture d'introduction (le) considérée à l'opposé du fond (1c).

Comme le montre la figure 5, la présence des soufflets permet d'augmenter, d'une manière significative, la contenance du sac permettant par conséquent d'y insérer des produits (P), tels que, par exemple, des plateaux d'épaisseur (e) relativement importante.

Comme indiqué dans la partie introductive de la description, l'emballage, selon les caractéristiques de l'invention, après avoir été rempli du ou des produits (P) et fermé hermétiquement, est disposé dans un autoclave, par exemple, pour un cycle de stérilisation à la vapeur qui entre et sort par la feuille (2).

D'une manière connue, l'emballage peut être équipé d'un témoin attestant du bon déroulement du cycle de stérilisation.

A noter également que le positionnement et la fixation de la feuille (2) sur les bords considérés du sac, s'effectuent par tout moyen connu et approprié du type de ceux déjà utilisés pour la fabrication de certains types de sacs.

Les avantages ressortent bien de la description.

## Revendications

1. Emballage pour la stérilisation à la vapeur de produits, sous forme d'un sac (1) en matière plastique dont les bords latéraux (1a) et (1b) sont conformés par pliage pour constituer des soufflets (a) et (b) formés par deux plis symétriques entre deux parois (1d) et (2), l'une des parois (2) au moins est réalisée dans une matière apte à laisser passer la vapeur pendant un cycle de stérilisation, **caractérisé en ce que** ladite paroi (2) est constituée par une feuille rapportée fixée sur les bords latéraux des soufflets considérés du côté du pli (b) et sur l'un des bords transversaux (1c) de l'autre paroi (1d) en matière plastique, en constituant le fond, ladite feuille (2) déborde de l'un des côtés latéraux du sac pour constituer une zone de préhension en vue de sa pelabilité.

2. Emballage selon la revendication 1, **caractérisé en ce que** la paroi (2) apte à laisser passer la vapeur, est fixée par une ligne de soudure déterminée pour permettre sa pelabilité.

3. Emballage selon l'une quelconque des revendications 1 à 2, **caractérisé en ce que** la partie ouverte (le) du sac, considérée à l'opposé de son fond (1c), est fermée d'une manière étanche, après mise en place du ou desdits produit(s) à l'intérieur dudit sac.

4. Emballage selon la revendication 1, **caractérisé en ce que** la paroi (2), apte à laisser passer la vapeur, est du papier.

5. Emballage selon la revendication 1, **caractérisé en ce que** la paroi (2), apte à laisser passer la vapeur, est un non tissé.

## Patentansprüche

1. Verpackung für die Dampfsterilisation von Produkten, in Form eines Beutels (1) aus Kunststoff, dessen längsseitige Ränder (1a) und (1b) derart durch einen Faltvorgang ausgestaltet sind, dass sie Bälge (a) und (b) darstellen, die von zwei symmetrischen Falten zwischen den Wänden (1d) und (2) gebildet werden, wobei mindestens eine der Wände (2) aus einem Stoff gefertigt ist, der dazu befähigt ist, während eines Sterilisationszyklus Dampf hindurchzulassen, **dadurch gekennzeichnet, dass** die Wand (2) aus einer angeformten Folie besteht, die an den längsseitigen Rändern der betreffenden Bälge seitens der Falte (b) sowie an einem der Querränder (1c) der anderen Wand (1d) aus Kunststoff, welche den Boden desselben bildet, befestigt ist, wobei die Folie (2) auf einer der längsseitigen Seiten des Beutels übersteht, um einen Griffbereich zu bilden, sodass sie abgezogen werden kann.

2. Verpackung nach Anspruch 1, **dadurch gekennzeichnet, dass** die Wand (2), welche dazu befähigt ist, Dampf hindurchzulassen, mittels einer bestimmten Schweißnaht befestigt ist, sodass sie abgezogen werden kann.

3. Verpackung nach einem beliebigen der Ansprüche 1 bis 2, **dadurch gekennzeichnet, dass** der offene Bereich (le) des Beutels, der als dem Boden (1c) gegenüberliegend angesehen wird, abdichtend verschlossen wird, nachdem das oder die Produkt(e) ins Innere des Beutels gegeben wurde(n).

4. Verpackung nach Anspruch 1, **dadurch gekennzeichnet, dass** die Wand (2), welche dazu befähigt ist, Dampf hindurchzulassen, aus Papier ist.

5. Verpackung nach Anspruch 1, **dadurch gekennzeichnet, dass** die Wand (2), welche dazu befähigt ist, Dampf hindurchzulassen, aus einem Vliesstoff ist.

## Claims

1. Packaging for sterilizing substances with steam, in the form of a bag (1) made of a plastics material and having side edges (1a) and (1b) shaped by folding to constitute gussets (a) and (b) formed by symmetrical folds between two walls (1d) and (2), at least one of the walls (2) being made of a material suitable for allowing steam to pass through it during a sterilization cycle, **characterized in that** said wall (2) is constituted by a separate sheet fastened to the side edges of the gussets at the folds (b), and to one of the transverse edges (1c) of the other wall (1d) made of a plastics material so as to constitute a bottom, said sheet (2) extending beyond one of the sides of the bag to constitute a graspable zone for enabling said sheet to be peeled off.

2. Packaging according to claim 1, **characterized in that** the wall (2) suitable for allowing steam to pass through it is fastened via a weld line that is determined to enable it to be peeled off.

3. Packaging according to any one of claim 1 to claim 2, **characterized in that** the open portion (1e) of the bag, opposite from its bottom (1c), is closed in sealed manner, after the substance(s) has/have been placed inside the bag.

4. Packaging according to claim 1, **characterized in that** the wall (2) suitable for allowing steam to pass through it is made of paper.

5. Packaging according to claim 1, **characterized in that** the wall (2) suitable for allowing steam to pass through it is made of a non-woven material.
